Europäisches Patentamt

(19) European Patent Office

Office européen des brevets

(11) Veröffentlichungsnummer: **0 052 841**
A1

## (12) EUROPÄISCHE PATENTANMELDUNG

(21) Anmeldenummer: **81109662.7**

(22) Anmeldetag: **13.11.81**

(51) Int. Cl.³: **A 61 M 25/00**

(30) Priorität: **20.11.80 DE 3043785**

(43) Veröffentlichungstag der Anmeldung:
**02.06.82 Patentblatt 82/22**

(84) Benannte Vertragsstaaten:
**AT BE CH DE FR GB IT LI LU NL SE**

(71) Anmelder: **INTERMEDICAT GMBH**
**Gerliswilstrasse 43**
**CH-6020 Emmenbrücke(CH)**

(72) Erfinder: **Glocker, Raymond, Dr.**
**Schlothweg 35**
**D-3508 Melsungen(DE)**

(74) Vertreter: **von Kreisler, Alek, Dipl.-Chem. et al,**
**Deichmannhaus am Hauptbahnhof**
**D-5000 Köln 1(DE)**

(54) **Zentralvenöser Katheter.**

(57) Katheter, insbesondere zur Einführung von Infusionslösungen (3), oder dergleichen in die Vene (2), bei dem in der Wand des Katheterschlauches (4) durchgehende seitliche Löcher (5,6 und 7) ausgebildet und einander paarweise in einer Ebene gegenüberliegend angeordnet sind.

FIG.1

EP 0 052 841 A1

VON KREISLER    SCHÖNWALD    EISHOLD    FUES    0052841
VON KREISLER    KELLER    SELTING    WERNER

PATENTANWÄLTE

Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

DEICHMANNHAUS AM HAUPTBAHNHOF

D-5000 KÖLN 1

B.Braun Melsungen AG
Melsungen

20.11.80          AvK/IM

## Zentralvenöser Katheter

-----------------------

Die Erfindung betrifft einen zentralvenösen Katheter,
insbesondere zur Zuführung von Infusionslösungen oder
dergl. in die Vene, bei dem in der Wand des Katheterschlauches
durchgehende seitliche Löcher ausgebildet sind.

Über einen zentralvenösen Katheter oder Cava-Katheter
werden häufig Infusionslösungen mit Osmolaritäten und
pH-Werten verschieden von denen des Blutes zugeführt.
Lokal kann es dabei zu hohen Konzentrationen der Lösungen
im Blut kommen. Geraten die roten Blutkörperchen in eine
hypotone Umgebung, so werden sie zuerst vollkommen sphärisch, verlieren dann den Inhalt mit niedrigem Molekulargewicht und dann, wobei ihre Membran platzt, das Hämoglobin.
Letzteres wird als Hämolyse bezeichnet. Ist die Lösung dagegen hyperton, so können Stechapfelformen entstehen.
Auch das Plasma kann durch die Infusionslösung geschädigt
werden. So könnten Eiweiße durch saures Milieu denaturiert werden. Das Blut aber fließt ständig am Katheter
vorbei und die infundierte Lösung wird spätestens im
rechten Herzen durch die dortigen Turbulenzen vollständig
mit dem Blut vermischt, wobei selbst bei voll geöffnetem
Tropfenregler des Infusionsgerätes die pro Zeiteinheit infundierte Lösungsmenge mehr als 100 mal kleiner ist als die

Telefon: (0221) 131041 · Telex: 888 2307 dopa d · Telegromm: Dompatent Köln

vorbeiströmende Blutmenge.

Das Blut ist also immer nur temporär, wenn auch zyklisch
wiederkehrend, der Belastung durch die Infusionslösung
ausgesetzt. Die Venenwand dagegen, auf die unter Umständen
eine hochkonzentrierte Lösung trifft, kann dadurch mehr
geschädigt werden als durch die mechanischen Belastungen,
die der Katheter bzw. seine Spitze ausüben.

Die erwähnten nachteiligen Erscheinungen bei einer Veneninfusion sind darauf zurückzuführen, daß bei üblichen
Kathetern nur eine einzige Ausströmöffnung vorhanden ist,
die sich an der Spitze des Katheterschlauches befindet und
durch seinen offenen Querschnitt gebildet wird. Auch Katheter mit seitlichen Löchern, die mit axialen Abständen einander einzeln gegenüberliegend angeordnet sind (DE-OS
29 27 788) beheben diese Nachteile nicht.

Der Erfindung liegt die Aufgabe zugrunde, einen zentralvenösen Katheter mit strömungsdynamisch optimiertem Ausgang zu schaffen, der eine schnelle und gleichmässige
Vermischung der Infusionslösung oder dgl. mit dem Blut
bewirkt.

Diese Aufgabe wird dadurch gelöst, daß die Löcher einander paarweise
in einer Ebene gegenüberliegend angeordnet sind. Die aus
den Lochpaaren des Katheterschlauches austretende Infusionslösung mischt sich außerordentlich schnell und
gleichmässig mit dem Blut. Es werden zu hohe lokale Konzentrationen der Lösungen im Blut vermieden und es ergibt
sich eine sehr schnelle Mischung der infundierten Flüssigkeit mit dem Blut. Eine Schädigung der Venenwand und
des Plasmas durch die Infusionslösung wird aufgrund des
strömungsdynamisch optimierten Ausganges vermieden.

Strömungstechnisch besonders vorteilhaft sind zwei oder

mehr Lochpaare, die mit axialem Abstand und umfangsmässig
zue9nander versetzt angeordnet sind. Dabei liegt jedes
Lochpaar von jedem weiteren Lochpaar höchstens wenige Millimeter entfernt, um eine optimale Wechselwirkung der seitlich austretenden Flüssigkeitsstrahlen zu gewährleisten.
Dies wird außerdem dadurch begünstigt, daß die Lochpaare
um etwa 90° kreuzweise umfangsmässig zueinander versetzt
sind, wobei die austretenden Strahlen eine schirmförmige
Strömungsfigur ausbilden.

Bei verschlossener Spitze des Katheterschlauches tritt
die Infusionslösung nur durch die seitlichen Lochpaare
aus dem Katheterschlauch in die Blutbahn aus. Vorteilhaft
ist das erste proximale Lochpaar mit Abstand zu der verschlossenen Spitze angeordnet und es ist der verschlossene Katheterschlauchabschnitt bis zum ersten proximalen
Lochpaar mit einer Füllung versehen, die aus Röntgenkontrastmaterial bestehen kann, damit der Katheter
sich besser positionieren lässt. Im übrigen soll die
Füllung einen Sumpf evtl. vorher aspirierten Blutes
verhindern.

Die axialen Abstände der Lochpaare können gleich oder ungleich sein. Auch die Durchmesser der Löcher verschiedener
Lochpaare können gleich oder ungleich sein.

Die Achse jedes Loches der Lochpaare kann in der Wand
des Katheterschlauches gerade oder schräg verlaufen, so
daß die Löcher der Infusionslösung eine bestimmte
Strömungsrichtung erteilen.

In der Zeichnung ist die Wirkungsweise des erfindungsgemässen Katheters durch Gegenüberstellung mit einem bekannten Katheter veranschaulicht.

Fig. 1      zeigt einen üblichen zentralvenösen Katheter mit offener Spitze und geschlossenem Schlauchmantel, der in eine im Längsschnitt dargestellte Vene eingeführt ist und

Fig. 2      veranschaulicht in entsprechender Darstellung einen erfindungsgemässen Katheter.

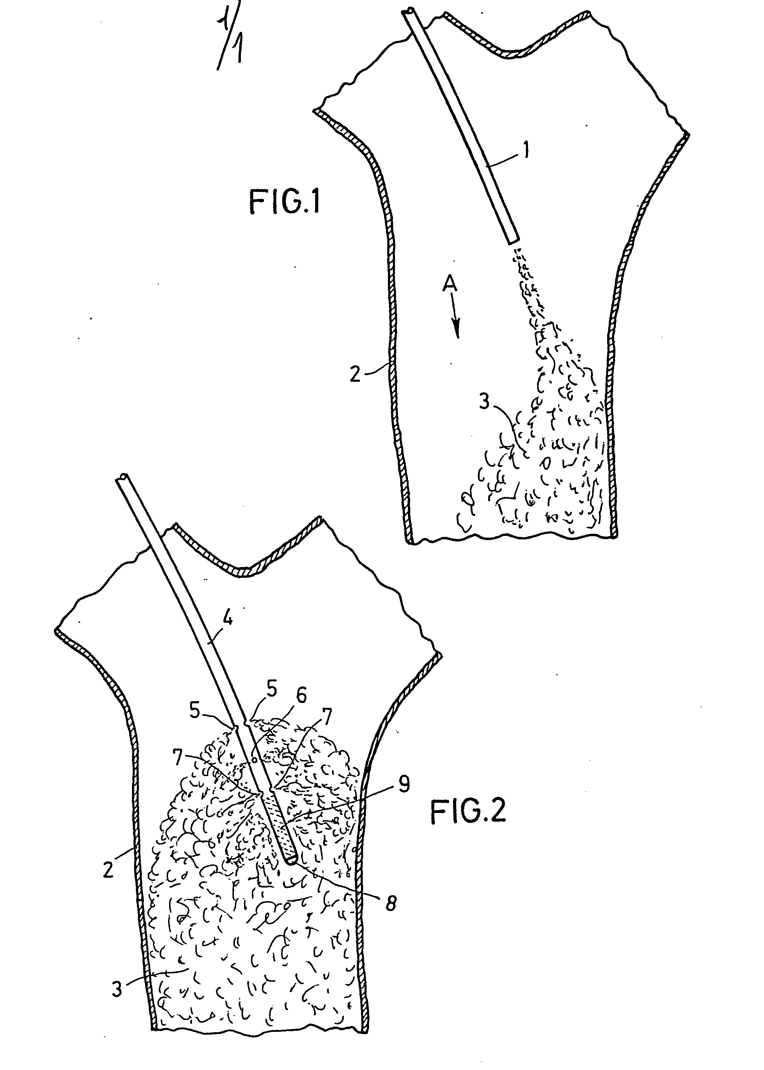

Ein bekannter Katheter 1 ist in eine Vene 2 eingeführt und es wird durch ihn eine Infusionslösung 3 in die Vene 2 infundiert. Die Infusionslösung 3 tritt aus der offenen Spitze des Katheters 1 in scharfem Strahl aus, der gegen die Wandung der Vene 2 gerichtet ist und sich an dieser Wandung konzentriert entlangbewegt. Das Blut kann in Richtung des Pfeils A an dem Infusionslösungs-Konzentrat vorbei strömen und selbst bei voll geöffnetem Tropfenregler des Infusionsgerätes ist die pro Zeiteinheit infundierte Lösungsmenge mehr als 100 mal kleiner als die vorbeiströmende Blutmenge. (Fig. 1).
Die Wand der Vene 2, auf die die unter Umständen hoch konzentrierte Infusionslösung 3 trifft, kann durch diese lokale Belastung trotzdem geschädigt werden.

Bei dem erfindungsgemässen zentralvenösen Katheter mit strömungsdynamisch optimiertem Ausgang ergeben sich völlig andere Verhältnisse, wie Fig. 2 zeigt. In der Wand eines Katheterschlauches 4 sind seitliche Löcher 5, 6 und 7 ausgebildet, die jeweils paarweise so angeordnet sind, daß sich zwei Löcher 5 bzw. 6 bzw. 7 diametral gegenüberliegen.

Das Lochpaar 6 ist zu den beiden Lochpaaren 5 und 7 kreuzweise, d.h. um 90° umfangsmässig versetzt.

Das erste proximale Lochpaar 7 befindet sich in einem gewissen Abstand zu der Spitze 8 des Katheterschlauches 4,
die verschlossen ausgebildet ist. Auf diese Weise ergibt sich
ein Abstandshalter, der bei versehentlicher wandständiger
Lage vorteilhaft ist. Das Schlauchstück zwischen der Katheterspitze 8 und dem Lochpaar 7 ist mit einer Füllung 9,
vorzugsweise aus röntgenkontrastgebenden Material, gefüllt.
Dieses hat die Aufgabe, die Positionierung des Katheters
zu erleichtern und einen Sumpf evtl. vorher aspirierten
Blutes in diesem verschlossenen Schlauchende zu verhindern.

Die Infusionslösung 3 tritt durch die Löcher 5, 6 und 7
seitlich aus dem Katheterschlauch 4 aus, verteilt sich
sofort über praktisch den ganzen Querschnitt der Vene 2
und vermischt sich dabei gleichmässig mit dem Blut. Die
Durchmischung findet also unmittelbar an den seitlichen
Ausgängen des Katheters statt, so daß schädliche lokale
hohe Konzentrationen der Infusionslösung im Blut nicht auftreten können und die Vene 2 von einer anteilig gleichmässigen Blut-Infusions-Lösungsmischung durchströmt ist.

Auch das Blut ist keiner Belastung durch lokale hohe
Konzentrationen der Infusionslösung ausgesetzt.

Die Abstände der Lochpaare 5, 6 und 7 sind bei dem dargestellten Beispiel gleich. Auch die Durchmesser der Löcher
der einzelnen Lochpaare sind gleich dargestellt. Bei anderen Ausführungsbeispielen können diese Faktoren zueinander ungleich sein, d.h. die Abstände der Lochpaare zueinander sind nicht identisch und die Löcher haben verschiedene Größen.

Patentansprüche
-----------------------------------

1.   Zentralvenöser Katheter, insbesondere zur Einführung von Infusionslösungen o.dgl. in die Vene, bei dem in der Wand des Katheterschlauches durchgehende seitliche Löcher ausgebildet sind, dadurch gekennzeichnet, daß die Löcher (5, 6, 7) einander paarweise in einer Ebene gegenüberliegend angeordnet sind.

2.   Zentralvenöser Katheter nach Anspruch 1, dadurch gekennzeichnet, daß mehrere Lochpaare (5,5; 6,6; 7,7) mit axialem Abstand und umfangsmässig zueinander versetzt angeordnet sind.

3.   Zentralvenöser Katheter nach Anspruch 2, dadurch gekennzeichnet, daß die Lochpaare (5,5; 6,6; 7,7) um etwa 90° kreuzweise umfangsmässig zueinander versetzt sind.

4.   Zentralvenöser Katheter nach den Ansprüchen 1 - 3, bei dem die Spitze des Katheterschlauches verschlossen ist, dadurch gekennzeichnet, daß das erste proximale Lochpaar (7,7) mit Abstand zu der verschlossenen Spitze (8) angeordnet und der verschlossene Katheterschlauchabschnitt bis zum ersten proximalen Lochpaar (7,7) mit einer Füllung (9) versehen ist.

5.   Zentralvenöser Katheter nach Anspruch 4, dadurch gekennzeichnet, daß die Füllung (9) aus Röntgenkontrastmaterial besteht.

6.   Zentralvenöser Katheter nach den Ansprüchen 1 - 5, dadurch gekennzeichnet, daß die axialen Abstände der Lochpaare (5,5; 6,6; 7,7) gleich oder ungleich sind.

7.   Zentralvenöser Katheter nach den Ansprüchen 1 - 6, dadurch gekennzeichnet, daß die Durchmesser der Löcher verschiedener Lochpaare (5,5; 6,6; 7,7) gleich oder ungleich sind.

8. Zentralvenöser Katheter nach den Ansprüchen 1 - 7, dadurch gekennzeichnet, daß die Achse jedes Loches (5, 6, 7) der Lochpaare in der Wand des Katheterschlauches (4) gerade oder schräg verläuft.

FIG.1

FIG.2

Europäisches Patentamt

**EUROPÄISCHER RECHERCHENBERICHT**

| **EINSCHLÄGIGE DOKUMENTE** | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | US - A - 3 128 769 (H.M. SCISLOWICZ) <br> * Anspruch; Spalte 1, Zeilen 9 bis 12; <br> Spalte 2, Zeilen 1 bis 7, 62 bis 66; <br> Fig. 2, 3 * <br> -- | 1-6 | A 61 M 25/00 |
| X | US - A - 3 828 767 (SPIROFF) <br> * Ansprüche 1 bis 3, 13; Spalte 1, <br> Zeilen 12 bis 19, 62 bis 67; Spalte 2, <br> Zeilen 1 bis 4; Spalte 3, Zeilen 3 bis <br> 20; Fig. 8 * <br> -- | 1,6-8 | |
| Y | US - A - 3 885 561 (CAMI) <br> * Anspruch 1; Spalte 2, Zeilen 3 bis 7, <br> 12 bis 22; Fig. 2 * <br> -- | 1,2,4, 5 | RECHERCHIERTE SACHGEBIETE (Int. Cl.³) <br><br> A 61 M 5/14 <br> A 61 M 25/00 |
| Y | DE - A - 1 933 802 (SHERWOOD MEDICAL <br> INDUSTRIES INC.) <br> * Seite 5, Zeilen 8 bis 15; Fig. 4, 5 * <br> -- | 1,2,4 | |
| D,Y | DE - A1 - 2 927 788 (H.G. WALLACE LTD.) <br> * Ansprüche 1 bis 3; Fig. 1 * <br> -- | 4 | |
| Y | DE - C - 2 016 607 (SHERIDAN) <br> * Spalte 5, Zeilen 49 bis 55; Fig. 9, <br> 10 * <br> -- <br><br> ./.. | 5 | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 05-02-1982 | CLOT |

EPA form 1503.1 06.78

**EUROPÄISCHER RECHERCHENBERICHT**

Europäisches
Patentamt

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| P,X | BIOMEDIZINISCHE TECHNIK, Vol. 26, Nr. 4 April 1981, Berlin R. GLOCKER "Strömungsdynamische Untersuchungen am Cavakatheter" Seiten 85 bis 92 * Seite 88, Abschnitt 6.2 c); Fig. 9. Seite 90, Spalte 8 * | 1-4 | |
| P,Y | GB - A - 2 065 480 (TECHNOLOGICAL SUPPLY S.A.) * Anspruch 1; Fig. 3 * | 4 | RECHERCHIERTE SACHGEBIETE (Int. Cl.³) |